(19) Europäisches Patentamt
European Patent Office
Office européen des brevets

(11) Numéro de publication : **0 335 776 B1**

(12) # FASCICULE DE BREVET EUROPEEN

(45) Date de publication du fascicule du brevet : **14.10.92 Bulletin 92/42**

(51) Int. Cl.$^5$ : **C07C 31/30,** C07C 29/70, C07C 29/76

(21) Numéro de dépôt : **89400809.3**

(22) Date de dépôt : **22.03.89**

(54) **Procédé de préparation de fluoroalcoolates de sodium.**

(30) Priorité : **29.03.88 FR 8804086**

(43) Date de publication de la demande : **04.10.89 Bulletin 89/40**

(45) Mention de la délivrance du brevet : **14.10.92 Bulletin 92/42**

(84) Etats contractants désignés : **AT BE CH DE ES FR GB GR IT LI LU NL SE**

(56) Documents cités : **EP-A- 0 159 020** **GB-A- 2 098 611**

(73) Titulaire : **ELF ATOCHEM S.A.**
**4 & 8, Cours Michelet La Défense 10**
**F-92800 Puteaux (FR)**

(72) Inventeur : **Pagniez, Guy**
**2, Bois de l'Eglise**
**F-64230 Poey de Lescar (FR)**
Inventeur : **Potin, Philippe**
**9, Rue des Pâquerettes**
**F-64140 Billère (FR)**

(74) Mandataire : **Rochet, Michel et al**
**ELF ATOCHEM S.A. Département Propriété Industrielle 4-8, Cours Michelet La Défense 10 - Cedex 42**
**F-92091 Paris-La-Défense (FR)**

EP 0 335 776 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un procédé amélioré de préparation de fluoroalcoolates de sodium.

Parmi les nombreuses applications des alcools fluorés, on peut citer la préparation de poly (fluoro alcoxy) phosphazènes, par réaction d'alcoolates de sodium sur du polydichlorophosphazène, selon le schéma :

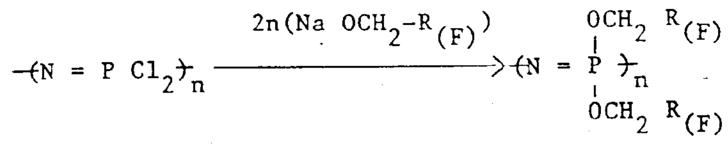


(voir par exemple le brevet US. 3.515.688).

Diverses méthodes ont été proposées pour préparer ces alcoolates de sodium, par réaction d'alcalinisation au moyen de sodium ou de composé du sodium, réaction appelée ci-après, par simplification, sodation.

Ainsi dans le brevet US 3.702.833, on propose de chauffer à reflux, dans du tétrahydrofuranne (THF), les alcools fluorés et du sodium métallique. Cette réaction conduit, à des degrés divers selon l'alcool utilisé, à une dégradation se traduisant par la formation de fluorure de sodium et l'observation d'une basicité du milieu inférieure à celle attendue, compte tenu de la quantité de sodium mise en jeu.

Le brevet US 4.357.458 propose d'effectuer la sodation au moyen de soude, grâce au déplacement d'équilibre provoqué par la distillation d'un hydrocarbure entraînant l'eau formée par la réaction.

Cette technique requiert un long temps de réaction (généralement supérieur à 20 heures).

Les brevets US 4.568.779 et 4.593.129 proposent de diviser finement le sodium avant sa mise en contact avec les alcools fluorés. On augmente ainsi la réactivité du sodium et on peut de ce fait opérer à basse température. Ce procédé nécessite toutefois l'emploi d'un réacteur équipé d'un système d'atomisation du sodium, d'un autre réacteur réfrigéré pour effectuer la sodation, la réaction de substitution étant elle-même réalisée dans un troisième appareil.

Il faut cependant noter que, même avec du sodium atomisé, la réaction reste longue lorsqu'elle est effectuée à basse température.

L'invention propose donc un procédé permettant en premier lieu d'effectuer une sodation rapide des alcools fluorés.

Elle propose également un procédé ne provoquant aucune dégradation des produits mis en oeuvre ou obtenus.

Elle propose encore un procédé permettant d'effectuer, dans le même réacteur, la réaction de sodation et la réaction de substitution de polydichlorophosphazène.

Ce procédé est caractérisé en ce qu'il consiste a/ à effectuer la sodation des alcools fluorés au moyen de méthylate de sodium au sein d'un solvant choisi dans le groupe constitué par les solvants susceptibles de présenter un point d'ébullition supérieur à celui du méthanol et/ou les solvants capables de former un azéotrope avec le méthanol, et b/ à piéger sélectivement le méthanol dégagé dans les vapeurs résultant de la mise à reflux de l'ensemble obtenu sous a).

Par solvant susceptible de présenter un point d'ébullition supérieur à celui du méthanol, on entend désigner tout solvant présentant cette propriété au moins à une certaine pression.

La séparation sélective du méthanol peut être réalisée au moyen d'un tamis moléculaire de porosité adéquate. A cette fin, on utilise avantageusement des tamis moléculaires de diamètre de pores d'environ 4 Angströms.

On effectue avantageusement les opérations suivantes :

1) Préparation du méthylate de sodium, par exemple à partir de sodium et de méthanol anhydre dans un solvant tel que défini ci-avant et qui peut être, par exemple, le THF, le diméthoxyéthane, le benzène, le cyclohexane, le dioxanne, le toluène.

Selon une variante, on peut préparer le méthylate de sodium dans un excès de méthanol, lequel est ensuite évaporé et remplacé par un solvant pouvant être choisi parmi les produits cités ci-avant ;

2) Introduction dans le même réacteur d'un alcool fluoré à bas point d'ébullition, seul ou en mélange avec d'autres alcools fluorés et/ou d'autres composés à hydrogène mobile tels que des alcools aliphatiques ou des phénols.

Les alcools fluorés peuvent appartenir aux différents produits de formule $R_{(F)}\ (CH_2)_n\ OH$, dans laquelle $R_{(F)}$ peut désigner sur un enchaînement hydrocarboné aliphatique ayant de 1 à 12 atomes de carbone, partiellement fluoré ou perfluoré, et n est égal à 1 ou 0. L'invention s'applique tout particulièrement aux mono, di et trifluoroéthanols.

3) Mise à ébullition de l'ensemble obtenu sous 2).

Les vapeurs sont constituées de méthanol, de l'alcool fluoré et du solvant. Selon une première variante (fig. 1) ces vapeurs traversent une colonne garnie de tamis moléculaire, par exemple de zéolithe 4A, sont condensées et redescendent dans le réacteur.

Selon une autre variante (fig. 2), les vapeurs se condensent directement et le condensat traverse de haut en bas la colonne de tamis moléculaire maintenue pleine au moyen d'un siphon.

Selon une troisième variante (fig. 3), les vapeurs se condensent directement et le condensat traverse de haut en bas ladite colonne de tamis moléculaire. Sur chacune des ces figures, 1 désigne le réacteur, 2 désigne la colonne de tamis moléculaire et 3 désigne le condenseur.

Dans tous les cas, le tamis moléculaire retient sélectivement le méthanol, l'alcool fluoré et le solvant retournant au réacteur.

La fin de la réaction peut être aisément contrôlée par l'absence de méthanol sous forme libre ou sodée dans le milieu réactionnel.

Indépendamment de l'absence totale de dégradation des réactifs et/ou des produits résultant de la réaction de sodation, le procédé conforme à l'invention présente un avantage remarquable dans la mesure où les fluoro-alcoolates de sodium auxquels il conduit peuvent être utilisés directement, dans le même réacteur, comme réactifs de substitution des polydichlorophosphazènes. Dans cette optique, on peut simplement introduire dans le milieu résultant des opérations précédentes le polydichlorophosphazène, avantageusement sous forme de solution dans un hydrocarbure tel qu'un hydrocarbure aromatique mono-ou polycyclique (benzène, naphtalène) ou un mélange pouvant renfermer de tels produits et soumettre l'ensemble aux conditions habituelles de cette réaction de substitution.

Les exemples suivants illustrent l'invention.

Dans ces exemples, l'ensemble des opérations est effectué sous pressurisation d'azote, avec des solvants et réactifs deshydratés. Le sodium est dépourvu de toute trace de soude.

EXEMPLE 1 (Comparatif)

On introduit dans un réacteur 26,22 g de trifluoroéthanol (0,262 mole), 24,55 g de trihydro 1,1′, oméga perfluoro alcanol-1 ($HCF_2\ (CF_2)_{2,4}\ CH_2\ OH$) (0,122 mole), 150 g de diéthylène glycoldiméthyl éther et 8,03 g de sodium (0,349 atome).

Le milieu est maintenu à 60°C. Le sodium disparaît ainsi en 2 heures. Au fur et à mesure de la disparition du sodium, un abondant précipité apparaît et le milieu réactionnel noircit.

La basicité théorique du milieu doit être de 0,349 équivalent. La basicité trouvée par dosage acidimétrique est seulement de 0,026 équivalent. 92,5 % du sodium a donc été consommé par réaction avec le fluor, ce que confirme l'analyse du précipité.

EXEMPLE 2 (Comparatif)

3,29 g de sodium (0,143 atome) sont finement divisés par une forte agitation dans 196 g de toluène à reflux. Dans ce milieu refroidi à 0°C, on introduit 43,85 g de trihydro-1,1′ oméga perfluoroalcanol-1 (0,217 mole).

Entre 0 et 10°C aucune réaction ne se manifeste.

Entre 10 et 20°C un faible dégagement d'hydrogène apparaît.

La réaction est maintenue pendant 24 heures à 25°C. La totalité du sodium a disparu et un précipité blanc est apparu.

Le dosage acidimétrique indique une basicité de 0,114 équivalent au lieu des 0,143 attendu, soit une perte de 20 %.

EXEMPLE 3

Une solution méthanolique de méthylate de sodium est préparée à partir de 180 g de méthanol et 3,40 g de sodium (0,147 atome).

Le méthanol est évaporé et remplacé par 300 ml de THF. On rajoute a cette solution, un mélange de 9,98 g de trifluoroéthanol (0,0997 mole), 9,61 g de trihydro 1,1′ oméga perfluoroalcanol-1 (0,0476 mole) et 1,50 g d'ortho-allylphénol (0,0108 mole).

Le réacteur est équipé d'un appareil correspondant au schéma 2 dont la colonne est garnie de 140 g de tamis moléculaire 4A.

L'ensemble est mis à reflux. Les vapeurs traversent ainsi la colonne de tamis moléculaire avant de revenir dans le réacteur. Au bout de 2 heures, aucune trace de méthanol n'est décelable dans le milieu réactionnel.

Auncun précipité n'est visible.

Le dosage acidimétrique indique 0,147 équivalent basique soit un rendement quantitatif en $R_F$ $(CR_2)$ ONa.

EXEMPLE 4

Une solution méthanolique de méthylate de sodium est préparée à partir de 20 g de méthanol et 2,135 g de sodium (0,0928 atome).

Le méthanol est évaporé et remplacé par 217,5 g de diméthoxyéthane.

On rajoute à cette solution 5,545 g de trifluoroéthanol (0,055 mole), 5,778 g de trihydro-1,1', oméga perfluoroalcanol-1 (0,029 mole) et 0,825 g d'orthoallylphénol (0,006 mole).

Le réacteur est équipé d'une colonne contenant 65 g de tamis 4A, d'un condenseur à reflux et d'un retour des condensats dans le réacteur selon le schéma 1.

Après 2 heures de reflux, le milieu réactionnel ne contient plus que 3 ppm de méthanol et aucune trace de précipité n'est visible.

Le dosage acidimétrique indique 0,093 équivalent basique, soit un rendement quantitatif en fluoroalcoolates de sodium.

EXEMPLE 5

On rajoute à la solution d'alcoolates obtenue dans l'exemple 4, une solution contenant 4,93 g de polydichlorophosphazène (0,085 équivalent chloré), 14,8 g de naphtalène et 14,8 g de benzène.

L'ensemble est chauffé pendant 12 heures à 70°C.

Le milieu réactionnel est concentré et redissous dans le minimum de THF, filtré sur terre d'infusoires et précipité dans le méthanol. Il est ensuite trituré dans le méthanol et mis à sécher sous vide (température 60°C ; pression réduite à 133 Pa).

On obtient ainsi 12,46 g de polymère se présentant sous forme de gomme, soit un rendement de 85 %, par rapport au polymère introduit.

EXEMPLE 6

Une solution méthanolique de méthylate de sodium est préparée à partir de 6 g de méthanol dans 200 g de diméthoxyéthane et 3,430 g de sodium (0,149 atome).

On rajoute à cette solution 8,720 g de trifluoroéthanol (0,087 mole), 8,438 g de trihydro-1,1', oméga perfluoroalcanol-1 (0,042 mole) et 4,508 g d'ortho-allylphénol (0,033 mole).

Le réacteur est équipé d'une colonne contenant 65 g de tamis 4A, d'un condenseur à reflux et d'un retour des condensats dans le réacteur (schéma 3).

Après 2 heures de reflux, le milieu réactionnel ne contient plus que 6 ppm de méthanol et aucune trace de précipité n'est visible.

Le dosage acidimétrique indique 0,150 équivalent basique, soit un rendement quantitatif.

EXEMPLE 7

On rajoute dans la solution d'alcoolates obtenue dans l'exemple 6, une solution contenant 7,83 g de polydichlorophasphazène (0,135 équivalent chloré), 23 g de naphtalène et 23 g de benzène.

L'ensemble est chauffé pendant 12 heures à 70°C.

Le mélange réactionnel est concentré et redissous dans le THF, filtré sur terre d'infusoires, concentré et précipité dans le méthanol. Il et ensuite trituré dans le méthanol et mis à sécher sous vide, (température 60°C ; pression réduite à 133 Pa).

On obtient ainsi 19,09 g de polymère, soit un rendement de 82 %.

## Revendications

1. Procédé de préparation de fluoroalcoolates de sodium, caractérisé en ce qu'il consiste a/ à effectuer l'alcalinisation des alcools fluorés au moyen de méthylate de sodium au sein d'un solvant choisi dans le groupe constitué par les solvants susceptibles de présenter un point d'ébullition supérieur à celui du méthanol et/ou les solvants capables de former un azéotrope avec le méthanol, et b/ à piéger sélectivement le méthanol dégagé dans les vapeurs résultant de la mise à reflux de l'ensemble obtenu sous a).

2. Procédé selon la revendication 1, caractérisé en ce que la séparation sélective du méthanol est effectuée sur tamis moléculaire.

3. Procédé selon la revendication 2, caractérisé en ce que le tamis moléculaire présente un diamètre de pore d'environ 4 Angströms.

4. Procédé selon l'une quelconque des revendications 1 à 3, caractérisé en ce qu'il consiste :
   a) a préparer le méthylate de sodium à partir de sodium et de méthanol anhydre dans un solvant tel que défini dans la revendication 1 ou à partir de sodium et d'un excès de méthanol, ensuite évaporé puis remplacé par un solvant tel que défini ci-avant,
   b) à introduire dans le dit milieu un ou plusieurs alcools fluorés, éventuellement en mélange avec d'autres composés à hydrogène mobile,
   c) à porter l'ensemble à ébullition et à séparer le méthanol par passage à travers une colonne garnie de tamis moléculaire.

5. Application des produits obtenus par la mise en oeuvre du procédé selon l'une quelconque des revendications 1 à 4 à la substitution de polydichlorophosphazènes.

## Patentansprüche

1. Verfahren zur Herstellung von Natriumfluoralkoholaten, gekennzeichnet durch folgende Schritte:
   a) Durchführung der Alkalialkoholatbildung der fluorierten Alkohole mit Hilfe von Natriummethylat in einem Lösungsmittel, das aus der Gruppe der Lösungsmittel, die einen Siedepunkt über dem von Methanol und/oder den Lösungsmitteln ausgewählt sind, die mit Methanol ein Azeotrop bilden können und
   b) selektiv das Methanol, in den beim Rückfluß des nach a) erhaltenen Gemisches anfallenden Dämpfen einfängt.

2. Verfahren nach Anspruch 1, dadurch gekennzeichnet, daß die selektive Abtrennung des Methanols mit einem Molekularsieb durchgeführt wird.

3. Verfahren nach Anspruch 2, dadurch gekennzeichnet, daß das Molekularsieb einen Porendurchmesser von etwa 4 Angström hat.

4. Verfahren nach einem der Ansprüche 1 bis 3, dadurch gekennzeichnet, daß es aus den folgenden Schritten besteht:
   a) Herstellung von Natriummethylat aus Natrium und wasserfreiem Methanol in einem entsprechend Anspruch 1 definierten Lösungsmittel oder aus Natrium und überschüssigem Methanol, das anschließend verdampft und durch das vorgenannte Lösungsmittel ersetzt wird,
   b) Zugabe von einem oder mehreren fluorierten Alkoholen gegebenenfalls im Gemisch mit anderen Verbindungen mit reaktionsfähigem Wasserstoff zu dem genannten Milieu,
   c) Erhitzen des Gemisches zum Sieden und Abtrennung des Methanols mittels Durchleiten durch eine mit dem Molekularsieb gefüllte Kolonne.

5. Verwendung der bei Einsatz des Verfahrens nach einem der Ansprüche 1 bis 4 erhaltenen Produkte für die Substitution von Polydichlorphosphazenen.

## Claims

1. Process for the preparation of sodium fluoroalcoholates, characterised in that it consists in a) carrying out the alkalination of the fluoroalcohols by means of sodium methylate in a solvent chosen from the group consisting of solvents capable of exhibiting a boiling point higher than that of methanol, and/or solvents capable of forming an azeotrope with methanol, and b) selectively trapping the methanol evolved in the vapours resulting from causing the combination obtained under a) to reflux.

2. Process according to Claim 1, characterised in that the selective separation of the methanol is effected on a molecular sieve.

3. Process according to Claim 2, characterised in that the meolecular sieve has a pore diameter of about 4 Angströms.

4. Process according to any one of Claims 1 to 3, characterised in that it consists in
a) preparing sodium methylate from sodium and anhydrous methanol in a solvent as defined in Claim 1 or from sodium and an excess of methanol which is then evaporated and thereafter by a solvent as defined above,
b) introducing into the said medium one or more fluoroalcohols, optionally mixed with other compounds possessing mobile hydrogen,
c) bringing the combination to the boil and separating the methanol by passage through a column packed with a molecular sieve.

5. Application of the products obtained by carrying out the process according to any one of Claims 1 to 4 to the substitution of polydichlorophosphazenes.

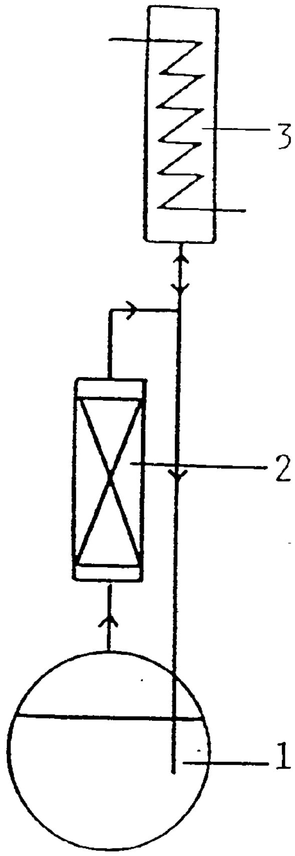


FIG. 1

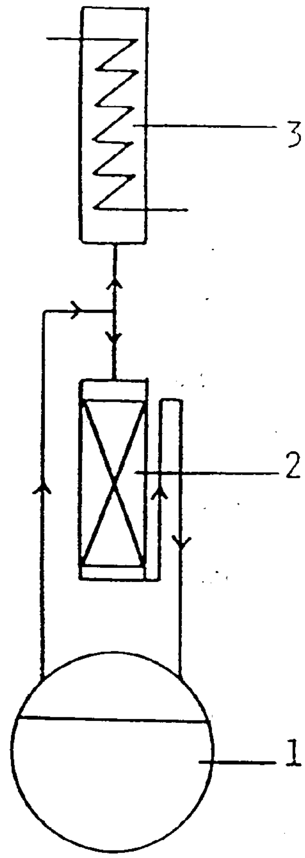


FIG. 2

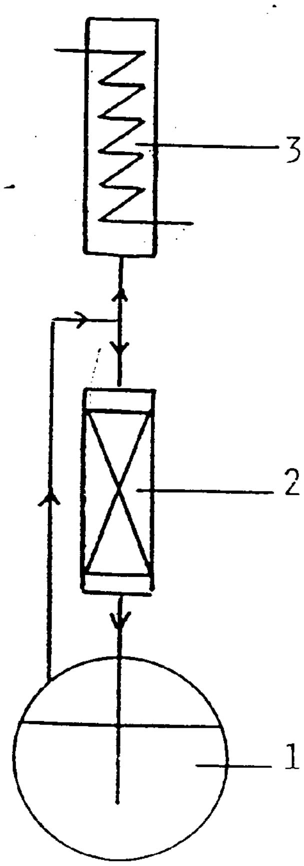


FIG. 3